(19) **Europäisches Patentamt**
**European Patent Office**
**Office européen des brevets**

(11) **EP 1 854 406 B1**

(12) **EUROPEAN PATENT SPECIFICATION**

(45) Date of publication and mention
of the grant of the patent:
**03.03.2010 Bulletin 2010/09**

(51) Int Cl.:
***A61B 5/06*** (2006.01)

(21) Application number: **07251969.7**

(22) Date of filing: **11.05.2007**

(54) **Position tracking of passive resonance-based transponders**

Verfolgung der Position von passiven Transpondern auf Resonanzbasis

Suivi de position de transpondeurs à résonance passive

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **12.05.2006 US 383020**

(43) Date of publication of application:
**14.11.2007 Bulletin 2007/46**

(73) Proprietor: **Biosense Webster, Inc.**
**Diamond Bar, CA 91765 (US)**

(72) Inventors:
• **Lichtenstein, Yoav**
**Raanana 43727 (IL)**
• **Levin, Michael**
**Haifa 35590 (IL)**

(74) Representative: **Tunstall, Christopher Stephen et al**
**Carpmaels & Ransford**
**43-45 Bloomsbury Square**
**London WC1A 2RA (GB)**

(56) References cited:
**US-A1- 2002 065 455     US-B1- 6 535 108**

## Description

## FIELD OF THE INVENTION

The present invention relates generally to position tracking systems, and particularly to methods and systems for tracking the position of passive transponders.

## BACKGROUND OF THE INVENTION

Various methods and systems are known in the art for tracking the coordinates of objects involved in medical procedures. Some of these systems use magnetic field measurements. For example, U.S. Patents 5,391,199 and 5,443,489, describe systems in which the coordinates of an intrabody probe are determined using one or more field transducers. Such systems are used for generating location information regarding a medical probe, such as a catheter. A position sensor is placed in the probe and generates signals in response to externally-applied magnetic fields. The magnetic fields are generated by magnetic field generators, such as radiator coils, fixed to an external reference frame in known, mutually-spaced locations.

Additional methods and systems that relate to magnetic position tracking are also described, for example, in PCT Patent Publication WO 96/05768, U.S. Patents 4,849,692, 4,945,305, 5,453,686, 6,239,724, 6,332,089, 6,618,612 and 6,690,963 and U.S. Patent Application Publications 2002/0065455 A1, 2003/0120150 A1, 2004/0068178 A1 and 2004/0147920 A1. These publications describe methods and systems that track the position of intrabody objects such as cardiac catheters, orthopedic implants and medical tools used in different medical procedures.

U.S. Patent 6,484,118, describes a medical tracking system and method for determining the position and orientation of an object utilizing a single axis position sensor and a position and orientation determination method.

Some transponders and sensors described in the patent literature comprise resonant circuits. For example, U.S. Patent 6,535,108, describes a transponder that has a resonant resistance-inductance-capacitance (RLC) circuit comprising one or more electromagnetic energy storage components that vary in response to an externally applied modulating energy field. In addition to the energy field, a base station transmits a carrier signal having a frequency essentially the same as the quiescent resonant frequency of the RLC circuit. As the components vary, the resonant frequency of the RLC circuit changes, modulating the carrier signal with the external modulating energy field. Effects of the modulation are detected by the base station. Information (e.g., the presence of a tag) is obtained by receiving and demodulating the modulated signal at the base station.

As another example, U.S. Patent 6,206,835, describes an implant device, which is responsive to an external interrogation circuit. The implant device includes a structure implantable within a living animal and operatively configured to carry out or assist in carrying out a function within the living animal. The device further includes an electrically passive sensing circuit for sensing a parameter associated with the function. The sensing circuit includes an inductive element, wherein the sensing circuit has a frequency dependent variable impedance loading effect on the interrogation circuit in response to an interrogation signal provided by the exciter/interrogator element, the impedance loading effect varying in relation to the sensed parameter.

Features of the present invention that are known from US 6, 535, 108 have been placed in the preamble of claims 1 and 10 appended hereto.

## SUMMARY OF THE INVENTION

Embodiments of the present invention provide methods and systems for tracking the position and orientation of a transponder attached to an object that is inserted into the body of a patient. In some embodiments, one or more field generators generate position-varying fields in a working volume comprising the transponder. The transponder comprises a resonant circuit whose resonance frequency varies in response to the ambient position-varying magnetic field in its vicinity. Thus, the resonance frequency is indicative of the position and orientation of the transponder with respect to the field generator that generated the field.

A position tracker remotely senses the resonance frequency of the resonant circuit in the transponder to determine the position and orientation of the transponder, and thus of the object, with respect to the one or more field generators.

In some embodiments, the resonant circuit comprises a field-responsive element, which changes its electrical properties responsively to the ambient magnetic field. In some embodiments, the field-responsive element comprises an inductor placed around a magneto-inductive core, which changes its magnetic permeability responsively to the position-varying magnetic field. The change in permeability changes the inductance of the inductor, which in turn changes the resonance frequency of the resonant circuit.

In other embodiments, the field-responsive element comprises a capacitor coupled to a magneto-restrictive element. The restriction and/or expansion of the magneto-restrictive element responsively to the position-varying magnetic field vary the capacitance of the capacitor and in turn the resonance frequency of the resonant circuit.

In some embodiments, transponders that use the configurations described herein are both passive and wireless, enabling them to remain operative for a substantially indefinite period of time without any wired connection to the external position tracking system.

In some embodiments, the methods and systems described herein can use magnetic fields having low frequencies, thereby enhancing the immunity of the system to distortion caused by metal objects in the magnetic field.

There is therefore provided, in accordance with an embodiment of the present invention, a system for position tracking, including:

> one or more field generators, which are arranged to generate one or more respective position-varying magnetic fields;
>
> a transponder including a resonant circuit having a resonance frequency and including a field-responsive element, which is operative to vary the resonance frequency responsively to the one or more magnetic fields; and
>
> a position tracker, which is arranged to remotely sense the resonance frequency of the resonant circuit and to determine a position of the transponder responsively to the sensed resonance frequency.

In an embodiment, the transponder is attached to an object adapted for insertion into a body of a patient, and the position tracker is arranged to determine a position of the object inside the body.

In another embodiment, the field-responsive element includes an inductor having an inductance, which varies responsively to the one or more magnetic fields so as to vary the resonance frequency. The inductor may include a core including a magneto-inductive material and having a magnetic permeability, which changes responsively to the one or more magnetic fields so as to vary the inductance.

In yet another embodiment, the field-responsive element includes a capacitor having a capacitance, which varies responsively to the one or more magnetic fields so as to vary the resonance frequency. In an embodiment, the capacitor includes conducting electrodes, and the field-responsive element includes a magneto-restrictive element attached to at least one of the electrodes and arranged to change a spatial relation of the electrodes responsively to the one or more magnetic fields so as to vary the capacitance.

In still another embodiment, each of the one or more field generators includes at least two field radiating coils driven by respective drive signals so as to generate a rotating magnetic field. Additionally or alternatively, the one or more field generators include two or more field generators at two or more different, respective locations, which are operated sequentially to generate respective position-varying magnetic fields.

In an embodiment, the position tracker is arranged to transmit a probe signal toward the transponder over a predetermined frequency range, to receive a signal produced by the transponder responsively to the probe signal, to measure a loading of the probe signal by the resonant circuit over the frequency range responsively to the signal produced by the transponder, and to estimate the resonance frequency responsively to the loading.

The present invention will be more fully understood from the following detailed description of the embodiments thereof, taken together with the drawings in which:

## BRIEF DESCRIPTION OF THE DRAWINGS

> Fig. 1 is a schematic, pictorial illustration of a position tracking system, in accordance with an embodiment of the present invention;
> Fig. 2 is a block diagram that schematically illustrates elements of a position tracking system, in accordance with an embodiment of the present invention; and
> Figs. 3A and 3B are block diagrams that schematically illustrate transponders in a position tracking system, in accordance with embodiments of the present invention.

## DETAILED DESCRIPTION OF EMBODIMENTS

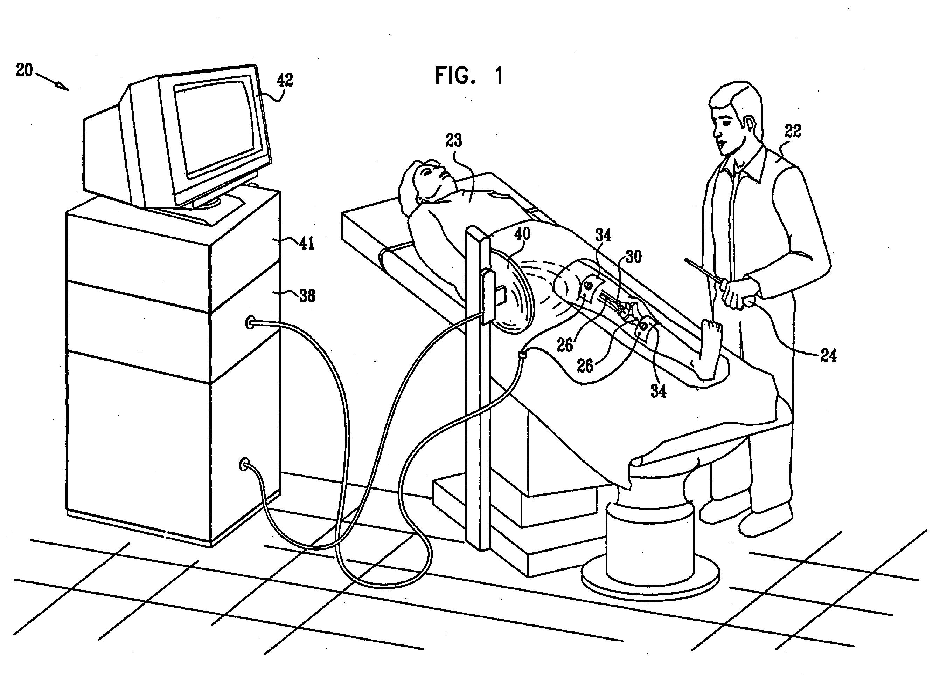

Fig. 1 is a schematic, pictorial illustration of a magnetic position tracking system 20 used in surgery, in accordance with an embodiment of the present invention. A surgeon 22 performs a medical procedure on a patient 23 using a medical tool 24. Implants 26 are introduced into the patient's body at a surgical site. In the present example the implants are placed in bones 30 of the patient's leg. System 20 guides the surgeon in performing the procedure, in this example a knee-joint operation, by measuring and presenting the positions of implants 26 and tool 24. The system measures the location and orientation coordinates throughout a working volume that comprises the surgical site.

The coordinates of tool 24 and implants 26 are determined relative to field generators, such as location pads 34, which are fixed to the patient's body. In the example shown in Fig. 1, the pads are placed on the patient's calf and thigh, in proximity to implants 26. A signal generator unit 38 generates drive signals that drive the field generators, typically comprising field generating coils, in location pads 34. The location pads are typically connected by wires to unit 38, although a wireless connection is also feasible. The field generating coils generate magnetic fields throughout the working volume, as will be explained in detail below.

Implants 26 and tool 24 contain miniature passive transponders. In principle, each transponder comprises a resonant circuit, whose resonance frequency varies in accordance with the ambient magnetic field in its vicinity. The magnetic fields generated by location pads 34 cause the resonant circuit fitted into each of tool 24 and implants 26 to have a certain resonance frequency, which depends on the ambient magnetic field and on the spatial orientation of the transponder with respect to the field.

A position tracker 40 remotely senses the resonance frequency of the resonant circuit of each transponder, and determines the position and orientation of the transponder responsively to the sensed resonance frequency. The results are typically presented to the surgeon on a display 42.

Although Fig. 1 shows a position tracking system used in orthopedic surgery, the systems and methods described herein can also be used in other position tracking applications such as cardiac imaging systems, as well as various non-medical applications. Depending on the application, similar transponders can be coupled to a catheter, an endoscope, a medical or surgical tool, or to any other suitable tracked object. Some exemplary systems that can use the methods and devices described herein are described in the above-cited publications. The methods and systems described hereinbelow are particularly suitable for applications in which it is desirable to have a passive transponder that has no wired interconnections with the external system. For example, when implanting a transponder into a bone, as in the orthopedic system of Fig. 1, it is advantageous for the transponder to be both passive and wireless. A passive transponder enables it to remain operative for a substantially unlimited period of time without the need for a surgical procedure to replace a battery. It is also advantageous for such a transponder to be wireless, since connecting wires to an orthopedic implant is cumbersome and increases the risk of infection and other complications.

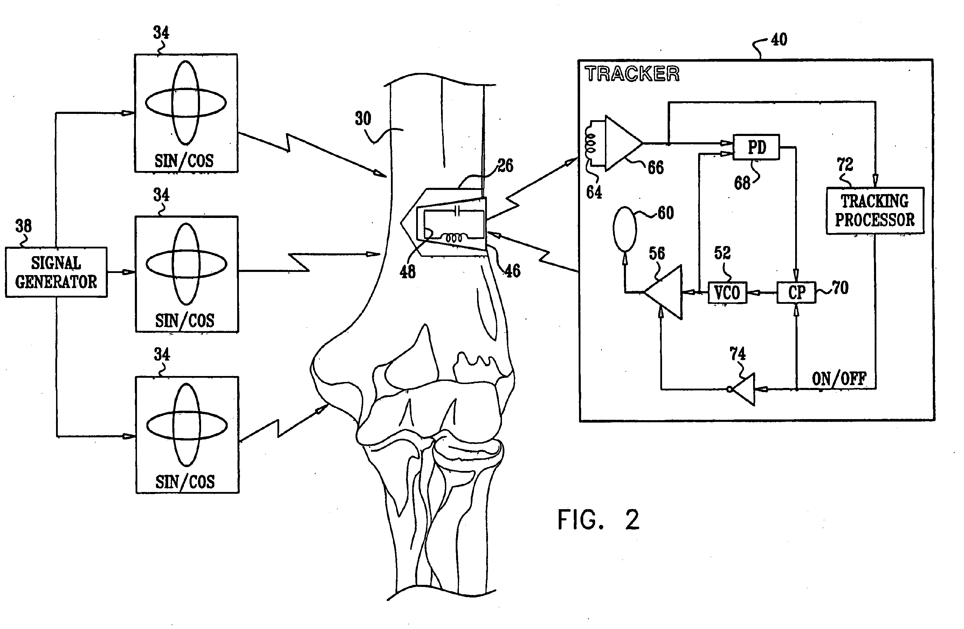

Fig. 2 is a block diagram that schematically illustrates elements of position tracking system 20, in accordance with an embodiment of the present invention. The figure shows implant 26 implanted into bone 30 of the patient. A passive transponder 46 is fitted into implant 26. A similar transponder can also be fitted into tool 24. Although the descriptions that follow refer to transponders fitted into implant 26, the methods and configurations described can also be used to track the position and orientation of tool 24.

In the exemplary configuration of Fig. 2, three location pads 34 generate position-varying magnetic fields in the vicinity of transponder 46. In some embodiments, each location pad 34 generates an alternating current (AC) magnetic field whose orientation changes (e.g., rotates) throughout the working volume.

A rotating magnetic field may be generated, for example, by configuring each location pad 34 to comprise two orthogonal field generating coils. The two coils are driven by signal generator unit 38 with two respective drive signals having the same frequency but different phases. The composite magnetic field produced by the two coils is in general an elliptically-rotating field, whose rotation pattern depends on the frequency and the relative phase and magnitude of the two drive signals. For example, equal magnitude drive signals having a 90 degree relative phase shift produce a circularly-rotating field. In alternative embodiments, the two coils may be non-orthogonal and/or be driven with drive signals having different frequencies. Further alternatively, any other suitable method can be used to generate the position-varying field by each location pad 34.

Unlike some position tracking systems that perform better when using drive signals (and magnetic fields) having relatively high frequencies, system 20 can be operated using magnetic fields having any convenient frequency. Using a low-frequency magnetic field is often desirable for improving the metal immunity of the system, i.e., reducing parasitic effects of metallic objects in the vicinity of the transponder, which distort the position measurement. A magnetic field having a frequency in the range of zero to several thousand Hertz is typically considered to be a low-frequency field, although other frequencies can also be used.

In some embodiments, when system 20 comprises two or more location pads 34, signal generator unit 38 drives the location pads one at a time in a time-division pattern. In general, there need not be a connection between the frequency, phase or magnitude of the magnetic fields generated by different location pads.

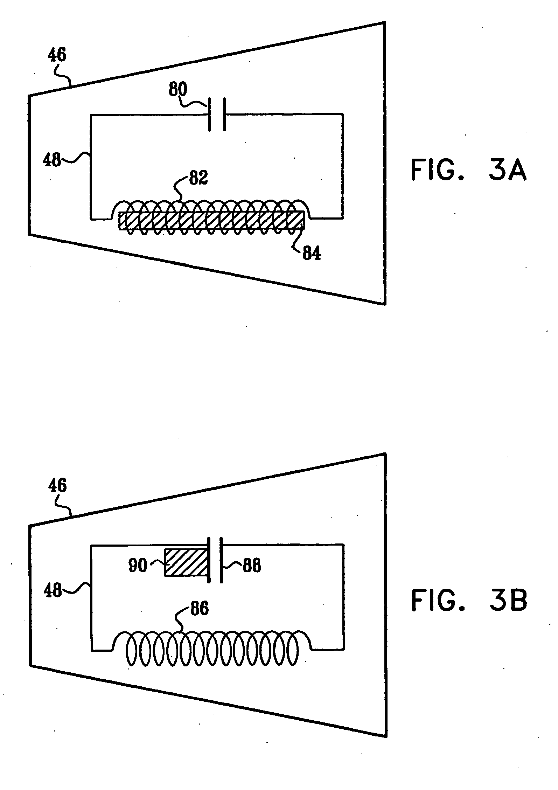

Transponder 46 in implant 26 senses the magnitude and/or the orientation of the magnetic field in its vicinity. Since the magnetic field produced by each location pad 34 is position-varying, the sensed magnetic field is indicative of the distance between implant 26 and the location pad 34 that generated the field. In some embodiments, transponder 46 comprises a resonant circuit 48, which is configured to change its resonance frequency in response to the ambient magnetic field. At each point in time, circuit 48 is located at a certain position and oriented at a certain spatial angle with respect to location pads 34. Resonant circuit 48 comprises at least one field-responsive component, which is operative, as will be explained in the descriptions of Figs. 3A and 3B below, to change its electrical properties responsively to the ambient magnetic field projected onto its axis. Thus, resonant circuit 48 changes its resonance frequency responsively to the component of the magnetic field projected on the axis of this component.

The resonance frequency of circuit 48 can be configured to any convenient value. In some embodiments, the resonance frequency is selected in the range between 100 kHz and 20 MHz. In one embodiment, the resonant circuit has a resonance frequency of approximately 135 kHz, a range commonly used in radio frequency identification (RFID) applications. Note that the resonance frequency of circuit 48 should not be confused with the frequency of the magnetic field. The two frequencies are generally unrelated and can be independently set to any suitable values.

Position tracker 40 remotely measures the resonance frequency of resonant circuit 48 in implant 26. Using the measured resonance frequency, tracker 40 estimates the position and orientation of implant 26 with respect to location pads 34. In embodiments in which location pads 34 are driven sequentially in a time-division pattern, the measurements of tracker 40 are typically synchronized with this pattern. In some embodiments, tracker 40 combines the measurements corresponding to the different location pads to determine the position and orientation coordinates of implant 26 with respect to the location pads. Tracker 40 can use various triangulation methods, as well as any other suitable method known in the art for combining the measurements into a position/ orientation estimate. The position/orientation estimate is often expressed as a six-dimensional coordinate of the implant. Additionally or alternatively, the position calculation can use single-axis methods, such as the methods described in U.S. Patent 6,484,118, cited above.

In an exemplary embodiment of tracker 40, a voltage controlled oscillator (VCO) 52 generates a probe signal that covers a predetermined frequency range. In some embodiments, the probe signal comprises a carrier, which is swept across the frequency range. Alternatively, the probe signal may comprise a carrier, which is hopped from one frequency to another in accordance with a predetermined frequency list. The probe signal may also comprise a wideband signal that instantaneously covers the frequency range or parts thereof. Further alternatively, any other method of scanning the predetermined frequency range can be used.

The probe signal is amplified by a transmit amplifier 56 and transmitted toward transponder 46 via a transmit antenna 60. Antenna 60 may comprise a transmission coil or any other suitable antenna configuration. In some embodiments, VCO 52 is set to the desired frequencies using a phase detector (PD) 68 and a comparator (CP) 70 arranged in a phase-locked loop (PLL) configuration, as is known in the art. A receive antenna, such as a reception coil 64, receives the signal produced by transponder 46 in response to the probe signal. The received signal is amplified by a receive amplifier 66 to produce a receiver output signal.

The probe signal induces current in resonant circuit 48. The amplitude of the induced current depends on the frequency of the probe signal with respect to the resonance frequency of circuit 48. In other words, the resonant circuit provides a different loading to the probe signal depending on the resonance frequency of the resonant circuit and the frequency of the probe signal.

The induced current causes the resonant circuit to produce an electromagnetic field, or signal, having the same frequency. The amplitude of the signal produced by the resonant circuit similarly depends on the frequency of the probe signal with respect to the resonance frequency of circuit 48.

When the frequency of the probe signal approaches the resonance frequency of circuit 48, the current induced in the resonant circuit approaches a maximum value, which in turn maximizes the amplitude of the signal received by the tracker. As a result, the tracker PLL locks on the resonance frequency of resonant circuit 48.

A tracking processor 72 performs the various measurement, analysis and control functions of tracker 40. Processor 72 may comprise a microprocessor running suitable software code. Alternatively, processor 72 may be implemented using suitable hardware or using a combination of hardware and software functions. In some embodiments, processor 72 controls VCO 52 and/or other components of tracker 40 to produce the probe signal at the desired frequency. Using the received signal, processor 72 analyzes the receiver output signal to estimate the resonance frequency of resonant circuit 48. Based on the estimated resonance frequency, processor 72 determines the position and orientation of transponder 46, and of implant 26, relative to location pads 34.

In some embodiments, tracking processor 72 sweeps the frequency of the probe signal produced by VCO 52 over the predetermined range and measures the magnitude of the received signal at each frequency. Processor 72 then estimates the resonance frequency of circuit 48 responsively to the measured received signal amplitudes across the frequency range. In some embodiments, processor 72 identifies the frequency having a maximum received signal magnitude. Alternatively, any other suitable method can be used by processor 72 to estimate the resonance frequency of circuit 48 based on the received signal.

In some embodiments, tracker 40 alternates between transmission and reception modes. In other words, the tracker transmits the probe signal at a particular frequency during a certain time interval, and then receives the signal produced by the transponder at a subsequent time interval. In these embodiments, tracking processor 72 may alternately switch the electrical power of CP 70 and transmit amplifier 56 on and off. When in transmit mode, transmit amplifier 56 is switched on and CP 70 is switched off, and *vice versa.* For example, in Fig. 2, an on/off control signal produced by processor 72 alternates the power between amplifier 56 and CP 70 using an inverter 74.

Fig. 3A is a block diagram that schematically illustrates an exemplary configuration of transponder 46, in accordance with an embodiment of the present invention. Resonant circuit 48 in this example comprises a capacitor 80 and an inductor 82, with inductor 82 serving as the field-responsive element. It is well known that the resonance frequency of circuit 48 is given by

$$f_r = \frac{1}{2\pi\sqrt{LC}},$$ wherein L denotes the inductance of inductor 82 and C denotes the capacitance of capacitor 80.

Inductor 82 is wound or otherwise placed around a core 84. The magnetic permeability of core 84, denoted $\mu$, affects inductance L of inductor 82, which in turn affects resonance frequency $f_r$ of circuit 48. In some embodiments, core 84 comprises a magneto-inductive material that varies its permeability responsively to the ambient magnetic field. The magneto-inductive material of core 84 may comprise, for example, a FeCuNbSiB/Cu/FeCuNbSiB film or similar material.

Alternatively, any other suitable material having magneto-inductive properties may be used for this purpose. Typically, the permeability of core 84 changes responsively to the component of the magnetic field projected along the axis of the core. Thus, the resonance frequency of circuit 48 is indicative of the position and orientation of implant 26 with respect to location pad 34 that generated the field.

Fig. 3B is a block diagram that schematically illustrates an exemplary configuration of transponder 46, in accordance with another embodiment of the present invention. In the configuration of Fig. 3B, circuit 48 is a parallel resonant circuit comprising an inductor 86 and a capacitor 88, with capacitor 88 serving as the field-responsive element. In some embodiments, capacitor 88 is coupled to a magneto-restrictive element 90, which constricts and/or expands responsively to the ambient magnetic field. Element 90 may comprise any suitable magneto-restrictive material known in the art, such as, for example, Terfenol-D.

In some embodiments, capacitor 88 comprises two co-facing conducting electrodes, such as conducting plates, separated by an air gap or a suitable dielectric. In the example of Fig. 3B, element 90 is mechanically attached to one of the conducting plates of capacitor 88. When the ambient magnetic field changes, element 90 constricts or expands, thereby changing the spatial relation between the electrodes, specifically by changing the width of the gap between the capacitor plates. The change in gap width changes the capacitance of capacitor 88, which in turn changes the resonance frequency of circuit 48. Typically, capacitor 88 and element 90 are arranged along an axis, so that the gap between the capacitor plates varies substantially only in response to constriction/expansion of element 90 along this axis. Thus, as in the configuration of Fig. 3A above, the resonance frequency of circuit 48 changes responsively to changes in component of the magnetic field along this axis.

In alternative embodiments (not shown in the figures), magneto-restrictive element 90 can change the spatial relation of the electrodes in other ways. For example, element 90 can be coupled to capacitor 88 so as to change the co-facing area of the conducting plates, which also affects the capacitance of capacitor 88. Further alternatively, capacitor 88 may comprise conducting electrodes having any shape or form, and magneto-restrictive element 90 may be coupled to the capacitor in any suitable way so as to vary the capacitance of the capacitor responsively to the ambient magnetic field. In alternative embodiments, a surface-acoustic-wave (SAW) device can also be used as the field-responsive element in resonant circuit 48, since the magnetic field changes the internal reflection behavior of the SAW device.

Although the methods and systems described herein mainly relate to position tracking by remote resonance frequency measurement of medical implants, tools and instruments, the principles of the present invention can also be used to perform position tracking in other applications, such as radio-frequency identification (RFID) tags and industrial magnetic field sensors.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art.

**Claims**

1. A system (20) for position tracking, comprising:

    one or more field generators (34), which are arranged to generate one or more respective position-varying magnetic fields;
    a transponder (46) comprising a resonant circuit (48) having a resonance frequency and comprising a field-responsive element, which is operative to vary the resonance frequency responsively to the one or more magnetic fields; and **characterised by**
    a position tracker (40), which is arranged to remotely sense the resonance frequency of the resonant circuit (48) and to determine a position of the transponder responsively to the sensed resonance frequency.

2. The system according to claim 1, wherein the transponder (46) is attached to an object adapted for insertion into a body of a patient, and wherein the position tracker (40) is arranged to determine a position of the object inside the body.

3. The system according to claim 1, wherein the field-responsive element comprises an inductor (82) having an inductance, which varies responsively to the one or more magnetic fields so as to vary the resonance frequency.

4. The system according to claim 3, wherein the inductor (82) comprises a core (84) comprising a magneto-inductive material and having a magnetic permeability, which changes responsively to the one or more magnetic fields so as to vary the inductance.

**5.** The system according to claim 1, wherein the field-responsive element comprises a capacitor (88) having a capacitance, which varies responsively to the one or more magnetic fields so as to vary the resonance frequency.

**6.** The system according to claim 5, wherein the capacitor (88) comprises conducting electrodes, and the field-responsive element comprises a magneto-restrictive element (90) attached to at least one of the electrodes and arranged to change a spatial relation of the electrodes responsively to the one or more magnetic fields so as to vary the capacitance.

**7.** The system according to claim 1, wherein each of the one or more field generators comprises at least two field radiating coils driven by respective drive signals so as to generate a rotating magnetic field.

**8.** The system according to claim 1, wherein the one or more field generators comprise two or more field generators at two or more different, respective locations, which are operated sequentially to generate respective position-varying magnetic fields.

**9.** The system according to claim 1, wherein the position tracker is arranged to transmit a probe signal toward the transponder over a predetermined frequency range, to receive a signal produced by the transponder responsively to the probe signal, to measure a loading of the probe signal by the resonant circuit over the frequency range responsively to the signal produced by the transponder, and to estimate the resonance frequency responsively to the loading.

**Patentansprüche**

**1.** System (20) zur Positionsverfolgung, umfassend:

einen oder mehrere Feldgenerator(en) (34), der/die zum Generieren von einem oder mehreren jeweiligen mit der Position variierenden Magnetfeld(em) gestaltet ist/sind; einen Transponder (46), der einen Schwingkreis (48) umfasst, der eine Resonanzfrequenz aufweist und ein auf ein Feld reagierendes Element umfasst, das betreibbar ist, um die Resonanzfrequenz als Reaktion auf das eine oder die mehreren Magnetfelder zu variieren; **gekennzeichnet durch** eine Positionsverfolgungseinrichtung (40), die zur Fernmessung der Resonanzfrequenz des Schwingkreises (48) und zur Bestimmung einer Position des Transponders als Reaktion auf die gemessene Resonanzfrequenz gestaltet ist.

**2.** System nach Anspruch 1, **dadurch gekennzeichnet, dass** der Transponder (46) an einem Objekt angebracht ist, das zum Einsetzen in den Körper eines Patienten geeignet ist, und dass die Positionsverfolgungseinrichtung (40) zur Bestimmung einer Position des Objekts innerhalb des Körpers gestaltet ist.

**3.** System nach Anspruch 1, **dadurch gekennzeichnet, dass** das auf ein Feld reagierende Element eine Spule (82) mit einer Induktivität umfasst, die als Reaktion auf das eine oder die mehreren Magnetfelder variiert, um die Resonanzfrequenz zu variieren.

**4.** System nach Anspruch 3, **dadurch gekennzeichnet, dass** die Spule (82) einen Kern (84) umfasst, der ein magnetoinduktives Material umfasst und eine magnetische Permeabilität aufweist, die sich als Reaktion auf das eine oder die mehreren Magnetfelder ändert, um die Induktivität zu variieren.

**5.** System nach Anspruch 1, **dadurch gekennzeichnet, dass** das auf ein Feld reagierende Element einen Kondensator (88) mit einer Kapazität umfasst, die als Reaktion auf das eine oder die mehreren Magnetfelder variiert, um die Resonanzfrequenz zu variieren.

**6.** System nach Anspruch 5, **dadurch gekennzeichnet, dass** der Kondensator (88) leitende Elektroden umfasst und das auf ein Feld reagierende Element ein magnetorestriktives Element (90) umfasst, das an mindestens einer der Elektroden angebracht ist und gestaltet ist, um eine räumliche Beziehung der Elektroden als Reaktion auf das eine oder die mehreren Magnetfelder zu ändern, um die Kapazität zu variieren.

**7.** System nach Anspruch 1, **dadurch gekennzeichnet, dass** jeder des einen oder der mehreren Feldgeneratoren mindestens zwei Feldabstrahlspulen umfasst, die von jeweiligen Steuersignalen angesteuert werden, um ein rotierendes Magnetfeld zu generieren.

**8.** System nach Anspruch 1, **dadurch gekennzeichnet, dass** der eine oder die mehreren Feldgeneratoren zwei oder mehr Feldgeneratoren an zwei oder mehr unterschiedlichen jeweiligen Orten umfasst, die sequentiell betrieben werden, um jeweilige mit der Position variierende Magnetfelder zu generieren.

**9.** System nach Anspruch 1, **dadurch gekennzeichnet, dass** die Positionsverfolgungseinrichtung gestaltet ist, um ein Sondensignal zum Transponder über einen vorab festgelegten Frequenzbereich zu senden, ein von dem Transponder als Reaktion auf das Sondensignal erzeugtes Signal zu empfangen, eine Belastung des Sondensignals durch den Schwingkreis über den Frequenzbereich als Reaktion auf das von den Transponder erzeugte Signal zu messen und die Resonanzfrequenz als Reaktion auf die Belastung zu schätzen.

## Revendications

1. Système (20) de suivi de position, comprenant :

⇒ un ou plusieurs générateurs de champs (34), qui sont agencés pour générer un ou plusieurs champs magnétiques variant en fonction de la position ;
⇒ un transpondeur (46) comprenant un circuit résonant (48) ayant une fréquence de résonance et comprenant un élément sensible aux champs, qui peut être utilisé pour modifier la fréquence de résonance en réponse auxdits un ou plusieurs champs magnétiques ; et **caractérisé par**
⇒ un dispositif de suivi de position (40), qui est agencé pour détecter à distance la fréquence de résonance du circuit résonant (48) et pour déterminer une position du transpondeur en réponse à la fréquence de résonance détectée.

2. Système selon la revendication 1, dans lequel le transpondeur (46) est fixé à un objet adapté pour être inséré dans un corps d'un patient, et dans lequel le dispositif de suivi de position (40) est agencé pour déterminer une position de l'objet à l'intérieur du corps.

3. Système selon la revendication 1, dans lequel l'élément sensible aux champs comprend une inductance (82) ayant une valeur d'inductance, qui varie en réponse auxdits un ou plusieurs champs magnétiques de manière à modifier la fréquence de résonance.

4. Système selon la revendication 3, dans lequel l'inductance (82) comprend un noyau (84) comprenant un matériau magnéto-inductif et ayant une perméabilité magnétique, qui varie en réponse auxdits un ou plusieurs champs magnétiques de manière à modifier l'inductance.

5. Système selon la revendication 1, dans lequel l'élément sensible aux champs comprend un condensateur (88) ayant une capacitance, qui varie en réponse auxdits un ou plusieurs champs magnétiques de manière à modifier la fréquence de résonance.

6. Système selon la revendication 5, dans lequel le condensateur (88) comprend des électrodes conductrices, et l'élément sensible aux champs comprend un élément magnétostrictif (90) fixé à au moins l'une des électrodes et agencé pour modifier une relation spatiale des électrodes en réponse auxdits un ou plusieurs champs magnétiques de manière à modifier la capacitance.

7. Système selon la revendication 1, dans lequel chacun desdits un ou plusieurs générateurs de champs comprend au moins deux bobines de rayonnement de champ commandées par des signaux de commande respectifs de manière à générer un champ magnétique tournant.

8. Système selon la revendication 1, dans lequel lesdits un ou plusieurs générateurs de champs comprennent deux générateurs de champs ou plus à deux emplacements respectifs différents ou plus, qui sont mis en oeuvre séquentiellement pour générer des champs magnétiques variant en fonction de la position respectifs.

9. Système selon la revendication 1, dans lequel le dispositif de suivi de position est agencé pour transmettre un signal de sonde vers le transpondeur dans une plage de fréquence prédéterminée, pour recevoir un signal produit par le transpondeur en réponse au signal de sonde, pour mesurer une charge du signal de sonde par le circuit résonant dans la plage de fréquence en réponse au signal produit par le transpondeur, et pour estimer la fréquence de résonance en réponse à la charge.

FIG. 1

EP 1 854 406 B1

FIG. 2

EP 1 854 406 B1

FIG. 3A

FIG. 3B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5391199 A
- US 5443489 A
- WO 9605768 A
- US 4849692 A
- US 4945305 A
- US 5453686 A
- US 6239724 B
- US 6332089 B
- US 6618612 B
- US 6690963 B
- US 20020065455 A1
- US 20030120150 A1
- US 20040068178 A1
- US 20040147920 A1
- US 6484118 B
- US 6535108 B
- US 6206835 B